# EUROPEAN PATENT SPECIFICATION

(11) **EP 3 565 826 B1**
(45) Date of publication and mention of the grant of the patent: **14.04.2021**
(21) Application number: 18701107.7
(22) Date of filing: 05.01.2018
(51) Int. Cl.: C07K 14/32, C07K 14/325, C12N 3/00, C12N 15/75, C12P 21/02, A01N 63/00, C12N 1/21

(54) **USE OF THE CPCR REGULATOR GENE FOR OBTAINING NEW RECOMBINANT STRAINS OF BACILLUS THURINGIENSIS WITH REDUCED SPORULATION CAPACITY**
VERWENDUNG DES CPCR-REGULATORGENS FÜR DIE ERHÖHUNG VON NEUEN REKOMBINANTEN STÄMMEN VON BACILLUS-THURINGIENSIS MIT REDUZIERTER SPORULIERUNGSKAPAZITÄT
UTILISATION DU GENE DE RÉGULATEUR CPCR POUR OBTENIR DE NOUVELLES SOUCHES RECOMBINANTES DE BACILLUS THURINGIENSIS AVEC CAPACITÉ DE SPORULATION RÉDUITE

(30) Priority: 05.01.2017 EP 17305011
(43) Date of publication of application: 13.11.2019
(73) Proprietor: Institut national de recherche pour l'agriculture, l'alimentation et l'environnement, 75007 Paris (FR); Institute of Plant Protection, Chinese Academy of Agricultural Sciences, Beijing 100193 (CN)
(72) Inventor: LERECLUS, Didier, 28260 Oulins (FR); SLAMTI, Leyla, 75015 Paris (FR); SONG, Fu Ping, Beijing 100193 (CN); DENG, Chao, Beijing 100193 (CN); ZHANG, Jie, Beijing 100193 (CN)
(74) Representative: Gevers & Orès
(86) International application number: PCT/EP2018/050288
(87) International publication number: WO 2018/127569

(56) References cited:
- QINGYUN FAN ET AL: "Regulation of sporulation by two-component system YvcPQ in Bacillus thuringiensis", ACTA MICROBIOLOGICA SINICA, vol. 57, no. 1, 19 July 2016 (2016-07-19), pages 121-130, XP055371099, DOI: 10.13343/j.cnki.wsxb.20160209
- YAO WANG ET AL: "Effect of quorum sensing response regulator nprR deletion on expression of Cry protein in Bacillus thuringiensis", ACTA MICROBIOLOGICA SINICA, vol. 50, no. 11, 16 June 2010 (2010-06-16) , pages 1550-1555, XP055371104, DOI: 10.13343/j.cnki.wsxb.2010.11.008
- ZHI-RU SONG ET AL: "Identification of similar transcriptional regulatory mechanisms in multiple cry genes in Bacillus thuringiensis HD12", JOURNAL OF INTEGRATIVE AGRICULTURE, vol. 16, no. 1, 1 January 2017 (2017-01-01), pages 135-143, XP055370370, AMSTERDAM, NL ISSN: 2095-3119, DOI: 10.1016/S2095-3119(16)61398-9
- CHAO DENG ET AL: "Regulation of cry Gene Expression in Bacillus thuringiensis", TOXINS, vol. 6, no. 7, 23 July 2014 (2014-07-23), pages 2194-2209, XP055368492, DOI: 10.3390/toxins6072194 cited in the application
- CHAO DENG ET AL: "Division of labour and terminal differentiation in a novel Bacillus thuringiensis strain", THE I S M E JOURNAL: MULTIDISCIPLINARY JOURNAL OF MICROBIAL ECOLOGY, vol. 9, no. 2, 1 August 2014 (2014-08-01), pages 286-296, XP055370359, United Kingdom ISSN: 1751-7362, DOI: 10.1038/ismej.2014.122 cited in the application
- GUIMING LIU ET AL: "Draft Genome Sequence of Bacillus thuringiensis Strain LM1212, Isolated from the Cadaver of an Oryctes gigas Larva in Madagascar", GENOME ANNOUNCEMENTS, vol. 2, no. 3, 29 May 2014 (2014-05-29), pages e00516-14, XP055370724, DOI: 10.1128/genomeA.00516-14
- MARK DE BEEN: "Comparative analysis of two-component signal transduction systems of Bacillus cereus, Bacillus thuringiensis and Bacillus anthracis", MICROBIOLOGY, vol. 152, no. 10, 1 October 2006 (2006-10-01), pages 3035-3048, XP055370700, GB ISSN: 1350-0872, DOI: 10.1099/mic.0.29137-0

## Description

### Field of the invention

The present invention relates to the use of a *cpcR* regulator gene for reducing the sporulation of a recombinant strain of *Bacillus thuringiensis,* a recombinant strain of *Bacillus thuringiensis* comprising the *cpcR* gene and uses thereof in particular as biopesticides.

### Background of the invention

### Bacteria and sporulation

*Bacillus* species are rod-shaped, endospore-forming aerobic or anaerobic, Gram-positive bacteria. The many species of the genus are able to live in every natural environment. Only one endospore is formed per cell. The spores are resistant to heat, cold, radiation, dessiccation, and disinfectants. At the onset of the sporulation, secondary metabolites are produced by *Bacillus sp..* These secondary metabolites may be: enzymes, antibiotics and insecticides.

*Bacillus larvae, Bacillus lentimorbus, Bacillus popilliae, Bacillus sphaericus,* and *Bacillus thuringiensis* are pathogens of specific groups of insects (Bacillus, Medical Microbiology). Thus, some *bacillus* are used as the active ingredients of insecticides.

*Bacillus thuringiensis* (Bt) is a bacterium present in soil, which produces spores and synthetize crystals releasing proteins which are toxic to insects and classified as Cryl, -2, -3, -4,..., according to their peptide sequences (Crickmore *et al.,* 1998). More particularly, Bt strains produce insecticidal crystals in the mother cell of sporulating bacteria which are used as biopesticides for controlling undesirable insects, such as Lepidoptera, Coleoptera and mosquitoes (Schnepf *et al.,* 1998). At the end of sporulation, the crystal is found alongside the spore. The promoters of genes encoding these crystal toxins are known. They are for example the promoters controlling the expression of genes *cry1, cry2, cry3, cry4* (Agaisse *et al.,* 1995; Deng *et al,,* 2014). The promoters of the *cry1, cry2 and cry4* genes are recognized by RNA polymerases associated to the specific sigma factors, Sigma E or Sigma K; the promoters of the *cry3* genes are recognized by RNA polymerase associated to the vegetative sigma factor, Sigma A. Fan *et al.,* 2017 discloses regulation of sporulation by two-component system YvcPQ in *Bacillus thuringiensis.* Wang *et al.,* 2010, discloses involvement of a response regulator, NprR, in *cry* gene expression in *Bacillus thuringiensis.* Song *et al.,* 2017, discloses transcriptional regulatory mechanisms in multipel *cry* genes in *Bacillus thuringiensis* strain HD12.

### Cry toxins

Cry toxins have specific activities against insect species of the orders Lepidoptera, Diptera, Coleoptera, Hymenoptera and against nematodes. When insects ingest crystals, their alkaline digestive tracts denature the crystals, making them soluble and thus amenable to being cut with proteases found in the insect gut, which liberate the toxin from the crystal (Schnepf *et al.,* 1998). The Cry toxin is then inserted into the insect gut cell membrane, paralyzing the digestive tract and forming a pore. The insect stops eating and starves to death (Schnepf *et al.,* 1998).

### Bt-based biopesticides

Bt is successfully used as a biopesticide for more than 50 years (Bravo *et al.,* 2011 and Sanahuja *et al.,* 2011). However, the efficiency of these Bt-based biopesticides is assessed to only a few days on the leaf surface. Indeed, the chemistry of the leaf surface, proteases and sunlight contribute to the degradation of Cry proteins.

Today, all the Bt-based biopesticides used in the world consist of a mixture of spores and crystals. However, toxins present in crystals are rapidly destroyed because they are released in the external medium at the end of the sporulation process. Further, the dissemination of bacterial spores may be detrimental for Human and environment.

It remains a need to provide safer Bt-based biopesticides wherein the stability of the Cry toxins is improved.

### Detailed description of the invention

In order to solve these problems, the inventors studied a Bt strain, called LM1212 that presents the unique ability to differentiate into crystal-producers or spore-formers. Transcriptional analysis suggested that the parasporal crystal phenotype results from a new type of cell differentiation associated with a novel regulation mode of *cry* gene expression (Deng *et al.,* 2015). As specified above, all biopesticides known until now consist of a mixture of spores and crystals. The characterization of the unusual LM1212 strain, in which the crystal is not produced in the mother cell of sporulating cells, but only in a subpopulation of non-sporulating cells, expanded inventor's understanding of the parasporal crystal phenotype in Bt. Thereby, the inventors have identified surprisingly and unexpectedly a unique regulatory sequence gene present in this LM1212 and called "crystal producing cells regulator" (*cpcR*). They showed that the introduction of this *cpcR* gene in a typical Bt strain drastically allows to reduce sporulation rate.

The inventors have characterized this regulator gene and its nucleotide sequence, which comprises the sequence SEQ ID NO: 1.

Thus, this CpcR regulator directs the expression of the *cry* gene promoters in *Bacillus thuringiensis* LM1212.

The inventors have also shown that the use of this regulator allows the expression of heterologous *cry* genes, as the *cry1Ab* gene encoding a lepidopteran active toxin, and the production of crystal inclusions in non sporulating Bt cells.

Moreover, the introduction of the *cpcR* gene in a typical Bt strain drastically reduced its sporulation rate while maintaining or increasing the expression of *cry* genes. The results obtained by the inventors indicate that this expression system allows the production of insecticidal Cry proteins in a Spo genetic background. Thus, the invention provides safer Bt-based biopesticides by preventing the dissemination of bacterial spores. Moreover, the production and the stability of the Cry toxins in dedicated bacterial cells are improved since the toxins are protected from the environmental degradation, such as UV irradiation.

Accordingly, the present invention relates to the use of the *cpcR* regulator gene of the sequence SEQ ID NO: 1 for reducing the sporulation of a recombinant strain of *Bacillus thuringiensis.*

The nucleotide sequence SEQ ID NO: 1 is:

"Recombinant strain" in the present invention means that one or more fragments of genes or one or more entire genes is/are inserted by genetic engineering, in a parental strain.

"Reducing the sporulation" in the present invention means that the rate of sporulation of the recombinant strain of Bt of the invention is reduced at least 2 fold preferably at least 5 fold, more preferably at least 10 fold, even more preferably at least 15 fold compared to the sporulation rate of the parental Bt strain.

In order to characterize the *cpcR* regulator gene, first the inventors determined a DNA fragment from LM1212, responsible for the activation of a promoter of a gene encoding a Cry protein in the LM1212. Then, they reduced this DNA fragment until identifying the gene responsible for this effect. After identification, the inventors validated that *cpcR* is responsible for a) the activation of *cry* gene expression in the LM1212 strain and b) the reduction of the sporulation rate. Finally, the inventors tested the use of the *cpcR* in a recombinant strain of Bt and obtained a reduction of the sporulation rate and an increase of the production of Cry1 Ab. These results confirm the interest to use the *cpcR* gene in a recombinant strain of Bt and the use of this recombinant strain of Bt as new biopesticide.

The *cpcR* regulator directs the expression of several promoters of genes encoding Cry and Cyt proteins, said promoters having the consensus sequence SEQ ID NO:2.

The nucleotide sequence SEQ ID NO: 2 is:
X₁TGAAX₂AAAAX₃X₄X₅X₆CAX₇X₈AX₉ATTTX₁₀CX₁₁TCX₁₂X₁₃X₁₄X₁₅X₁₆TX ₁₇X₁₈AX₁₉ATGTX₂₀X₂₁TX₂₂GX₂₃TAX₂₄AX₂₅TX₂₆X₂₇X₂₈X₂₉AX₃₀X₃₁TX₃₂X₃₃, with:
X₁=A or G; X₂ =C or T; X₃=A or T; X₄=A, T or G; X₅=A, C or T; X₆=A or G; X₇=C or T; X₈=A or C X₉=A, T or G; X₁₀=A or C; X₁₁=A or C; X₁₂=A, C or T; X₁₃=A, G or T; X₁₄=A or C; X₁₅=A, G or T; X₁₆=A or G; X₁₇=A or T; X₁₈=A, C or T; X₁₉=C or T; X₂₀=A or C; X₂₁=A, C or G; X₂₂=A or T; X₂₃=C or T; X₂₄=G or T; X₂₅=C or T; X₂₆=G or T; X₂₇=A or G; X₂₈=A or T; X₂₉=A, G or T; X₃₀=C, G or T; X₃₁=A or G; X₃₂=A or G; X₃₃=C or T.

Preferably, the promoter activated by CpcR, is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID: 6.

More preferably, the promoter activated by CpcR is P₃₅.

According to the invention, genes encoding the proteins activated by CpcR in the LM1212 strain, are preferably *cry* genes, more preferably *cry32Va1, cry41Ca1, cry45Ba1, cry74Aa1, cry32Wa1* and *cry35-like,* even more preferably *cry32Wa1* and *cry35-like.*

According to the invention, genes encoding proteins, in particular toxins, produced by the recombinant strain of *Bacillus thuringiensis* are preferably *cry* genes or *cyt* genes.

Preferably, the *cry* and *cyt* genes encoding toxins produced by the recombinant strain of the invention under the control of CpcR are:
1) *cry* genes encoding toxins active against crop pests, specifically lepidopteran and coleopteran insects. These *cry* genes belong for example to the classes *cry1, cry2, cry3, cry8* or *cry9.*
2) *cry* genes encoding toxins active against insect vectors of human diseases, specifically dipteran insects as mosquitoes and simuli. These *cry* genes belong for example to the classes *cry4* and *cry11.*
3) *cry* genes encoding toxins active against nematodes. These *cry* genes belong for example to the classes *cry5, cry6, cry14* and *cry21.*
4) *cyt* genes encoding toxins active against insect vectors of human diseases, specifically dipteran insects as mosquitoes and simuli. These *cyt* genes belong for example to the classes *cyt1* and *cyt2.*

More preferably, the genes encoding toxins produced by the recombinant strain of the invention under the control of CpcR are *cry1, cry2, cry3, cry4, cry5, cry 6, cry8, cry9, cry11, cry14, cry21, cyt1* or *cyt2* genes.

The activity spectrum of the Cry and Cyt toxins against insect pests is described in: Van Frankenhuizen, K., *et al.,* 2009. The activity spectrum of the Cry toxins against nematodes is described in: Wei *et al.,* 2003.

More preferably, the toxin produced by the recombinant strain of the invention is CrylAb, a lepidopteran active toxin, encoded by the *cry1Ab* gene.

The inventors showed that the expression of the *cpcR* gene allows the expression of heterologous *cry* genes, such as the *cry1Ab* gene, and the production of crystal inclusions in non-sporulating Bt cells.

The present invention also relates to a recombinant strain of *Bacillus thuringiensis,* which is characterized in that it comprises:
a) At least one gene encoding Cry and/or Cyt toxins,
b) At least one promoter having the sequence SEQ ID NO: 2, allowing the expression of said at least one gene encoding Cry and/or Cyt toxins, and,
c) A *cpcR* regulator gene of sequence SEQ ID NO: 1, which directs the expression of said at least one promoter.

The main feature of this recombinant strain, in the present invention, is its reduced sporulation rate in comparison with the parental strain.

According to the invention, this bacterium may be used living or dead. Indeed, the viability of the bacteria is not required to ensure the insecticidal or the nematicidal activity of this recombinant strain of *Bacillus thuringiensis.* Since CpcR negatively affects sporulation, the number of viable spores will be reduced or even abolished.

Preferably, the parental strain of *Bacillus thuringiensis* is the *Bacillus thuringiensis kurstaki* HD73 (type strain of the serotype 3a, 3b, 3c).

Preferably, the promoter present in the recombinant strain of *Bacillus thuringiensis* is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID: 6.

More preferably, the promoter present in the recombinant strain of *Bacillus thuringiensis* is P₃₅.

According to the invention, the promoter may be cloned on a plasmid, which is different to the plasmid carrying the *cpcR* regulator gene or the promoter may be cloned on the same plasmid as the *cpcR* regulator gene.

More preferably, the promoter is cloned on the same plasmid as the *cpcR* regulator gene.

According to the invention, genes encoding the toxins in the recombinant strain of *Bacillus thuringiensis* are preferably *cry* or *cyt* genes, more preferably *cry1, cry2, cry3, cry4, cry5, cry6, cry8, cry9, cry11, cry 14, cry 21, cyt1* or *cyt2 genes,* more preferably, *cry1, cry2, cry3, cry8 or cry9* genes.

Preferably, the toxins produced by the recombinant strain of the invention are Cry1 and Cry2 toxins.

More preferably, the toxin produced by the recombinant strain of the invention is Cry1Ab, a lepidopteran active toxin, encoded by the *cry1Ab* gene.

The present invention also relates to the use of the recombinant strain of *Bacillus thuringiensis* as described above as biopesticide, wherein said use is not a method for treatment of the human or animal body by therapy.

"Biopesticide" in the present invention refers to pesticides derived from natural materials, such as bacteria, for example, in the present invention. This general term "biopesticide" includes the term "biocide" which is more used in a context of controlling vectors, such as vectors that transmit pathogens responsible for mammalian diseases.

In a particular embodiment, the recombinant strain of *Bacillus thuringiensis* is used for protecting cultures. The "biopesticide" producing CpcR-regulated Cry proteins will be used for controlling crop pests, specifically lepidopteran insects such as those belonging to the families *Tortricidae, Noctuidae* and *Pyralidae* which damage crops like cotton, cabbage, com, soybean, grapevine and any other extensive or gardener cultures.

Preferably, the toxins produced by the recombinant strain of the invention for protecting cultures are: Cry1, Cry2, Cry3, Cry 8 and Cry9 toxins.

In another particular embodiment, the recombinant strain of *Bacillus thuringiensis* is used to control vectors, especially vectors that transmit pathogens responsible for mammalian diseases, preferably human diseases, such as insect vectors, in particular mosquitoes or 2. simuliid. In such a particular embodiment, the recombinant strain of *Bacillus thuringiensis* will harbor CpcR-regulated *cry* genes encoding toxins specifically active against dipteran insects.

Preferably, the toxins produced by the recombinant strain of the invention in the use to control vectors are toxins belonging, for example, to the classes Cyt1, Cry4 and Cry11.

In another particular embodiment, the recombinant strain of *Bacillus thuringiensis* is used to control nematodes, especially those responsible for mammalian diseases, preferably human diseases. In such a particular embodiment, the recombinant strain of *Bacillus thuringiensis* will harbor CpcR-regulated *cry* genes encoding toxins specifically active against nematodes.

Preferably, the toxins produced by the recombinant strain of the invention in the use to control nematodes are toxins belonging, for example, to the classes Cry5, Cry6, Cry14 and Cry21.

The present invention also relates to a method for obtaining a recombinant strain of *Bacillus thuringiensis* as described above, comprising the steps of introducing in said strain both:
1- a genetic construction comprising at least one gene encoding a toxin under the control of a promoter having the sequence SEQ ID NO: 2, and
2- an expression system comprising the *cpcR* regulator gene of the sequence SEQ ID NO: 1.

The two steps of this method can be performed in the order as defined above or in the reverse order but also simultaneously by introducing a construct carrying the three cited elements.

Preferably, the genetic construction as defined in step 1 of said method and the expression system as defined in step 2 of said method are on the same plasmid. In such an embodiment, step 1 and step 2 can be performed simultaneously.

Preferably, the parental strain of *Bacillus thuringiensis* is the *Bacillus thuringiensis kurstaki* HD73.

Preferably, the promoter having the sequence SEQ ID NO: 2 is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID: 6.

More preferably, said promoter is P₃₅ of sequence SEQ ID NO: 2.

Preferably, the genes encoding a toxin are *cry1, cry2, cry3, cry 4, cry5, cry6, cry8, cry9, cry11, cry14, cry21* or *cyt* genes, more preferably *cry1, cry2, cry3, cry8* or *cry9 genes.*

More preferably, the gene encoding a toxin is *cry1Ab* gene.

The promoter which directs the transcription of the *cpcR* gene is any promoter functional in Bt. The choice of the more appropriate promoter may depend in particular on the type of expression (*i.e.* constitutive or inducible) that one wishes to obtain. Promoters may be constitutive promoters, *i.e*. promoters which are active in cells and under most environmental conditions, or cell specific promoters which are active only or mainly in certain cell types, and inducible promoters that are activated by physical or chemical stimuli.

The genetic construction will be done on Bacillus plasmids based on the replicons pBC16, pHT315 or pHT73 and carrying a *cry* gene downstream from a CpcR-regulated promoter.

The genetic construct can be introduced in a strain of *Bacillus thuringiensis* by electroporation (Lereclus *et al.,* 1989; Bone *et al.* 1989; Koehler *et al.* 1994; Mahillon *et al.,* 1999; Peng *et at.* 2009), by using recombination procedure as previously described (Lereclus *et al.,* 1992), or by heterogramic conjugation (Trieu-Cuot *et al.,* 1987).

In addition to the above features described, the invention further comprises other features which will emerge from the following description, which refers to examples illustrating the present invention, as well as to the appended figures.

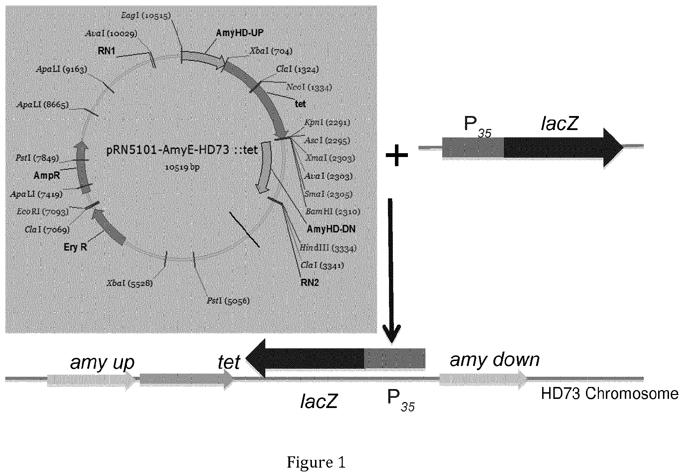

**Figure 1** shows the construction of a *Bacillus thuringiensis* strain.

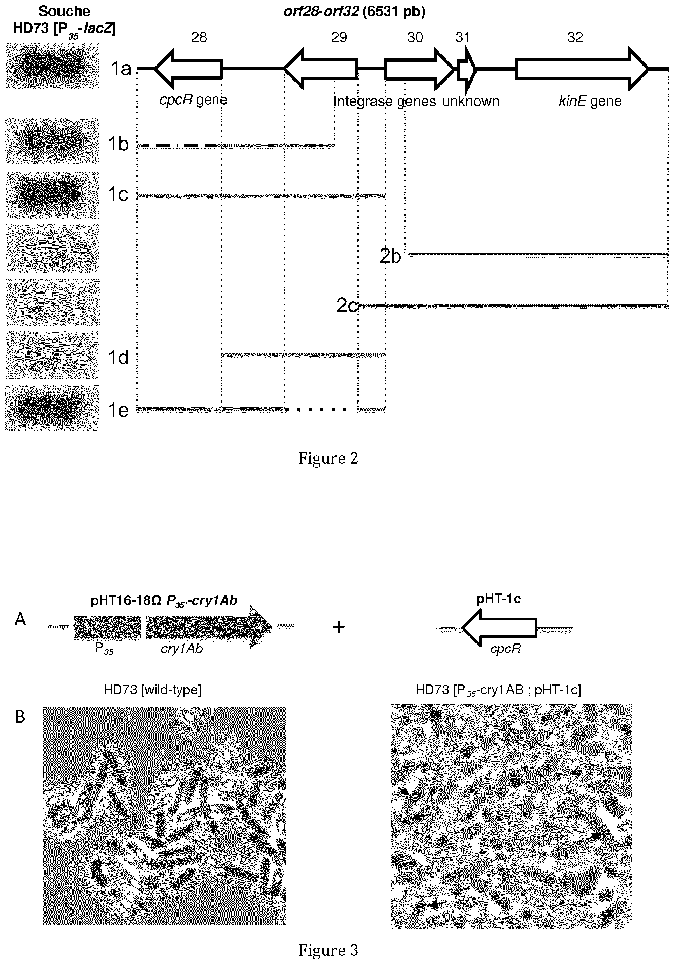

**Figure 2** shows the characterization of the LM1212 DNA fragment carrying the *cpcR* gene activating transcription from the P₃₅ promoter.
**Figure 3** shows the expression of *cry1Ab* under the control of CpcR and P₃₅ in a *kurstaki* strain. A) Plasmids pHT16-18ΩP35'-*cry1Ab* and pHT-1c. B) Phase-contrast microscopy of the strain *kurstaki* HD73 transformed with plasmids pHT16-18ΩP35'-*cry1Ab* and pHT-1c. The arrows indicate some crystal inclusions.

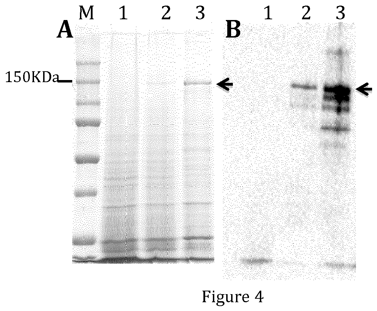

**Figure 4** shows the analysis of a Cry1Ab toxin produced in a *kurstaki* strain under the control of CpcR and P₃₅, A) SDS-Page. M corresponds to molecular weight markers. 1. 20 µL of crude extract from colonies of the strain *kurstaki* HD73 (pHT16-18, pHT304). 2. 20 µL of crude extract from colonies of the strain *kurstaki* HD73 (pHT16-18ΩP35'-*cry1Ab*, pHT304). 3. 20 µL of crude extract from colonies of the strain *kurstaki* HD73 (pHT16-18ΩP35'-*cry1Ab*, pHT-1c). The crude extracts were prepared in the same way and the use of 20 µL of crude extract means that the same amount of each preparation is loaded on the SDS-Page. B) Western blotting of lanes 1, 2 and 3 with antisera against Cry1Ab.

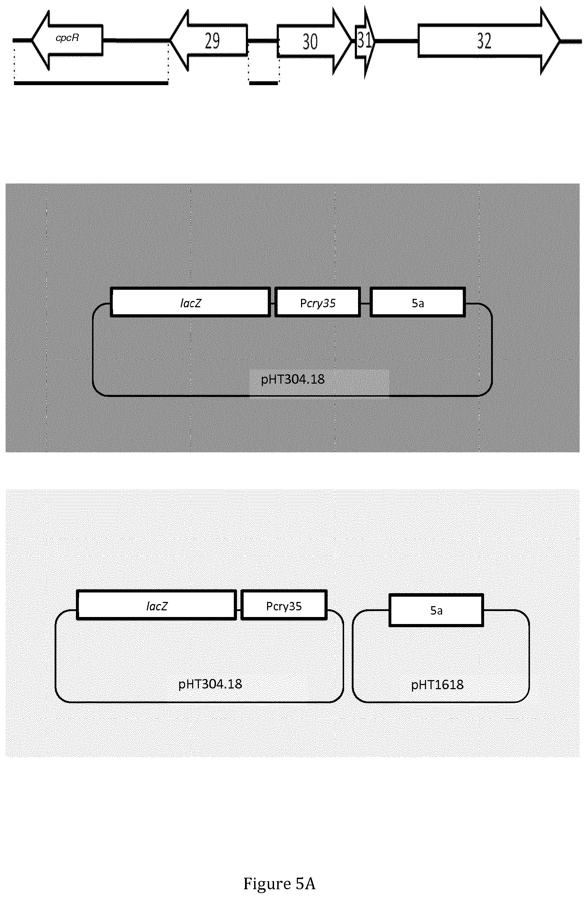

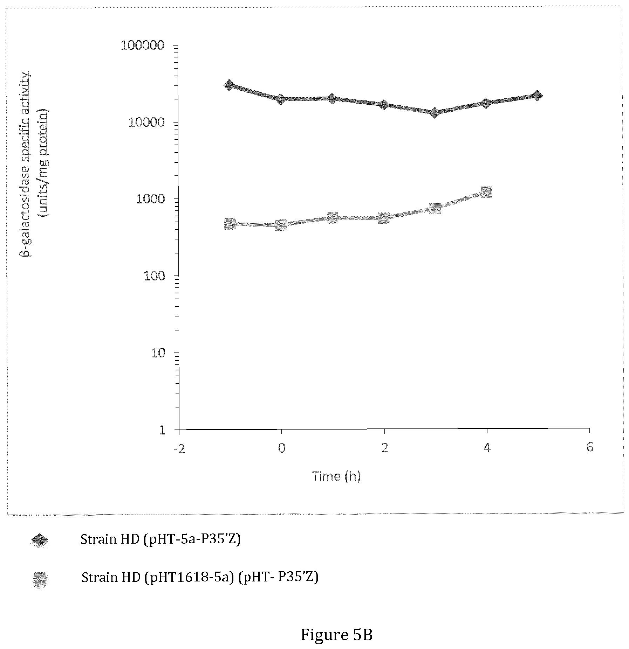

**Figure 5** shows the transcriptional analysis of the P35'lacZ fusion. A. Detail of the constructs. The upper panel shows the DNA fragment 5a carrying the *cpcR* gene. The two lines below the schematic representation of the *cpcR* locus indicates the DNA regions used to create fragment 5a. The middle panel highlighted in dark grey shows the organization of plasmid (pHT-5a-P35'Z) and the lower panel highlighted in light grey shows the organization of plasmids (pHT1618-5a) and (pHT- P35'Z). B. β-galactosidase assay of strains HD (pHT-5a-P35'Z) in dark grey lozenges and HD (pHT1618-5a) (pHT-P35'Z) in light grey squares. The time indicated is relative to t0 which indicates the beginning of the transition between exponential and stationary phase.

### EXAMPLE 1: CONSTRUCTION OF A BACILLUS THURINGIENSIS STRAIN TO SCREEN FOR THE REGULATOR OF THE EXPRESSION FROM THE P₃₅ PROMOTER

The previous results obtained by the inventors suggested that, in the LM1212 strain, a regulation system limits the production of toxins to the subpopulation of non-sporulating cells. Thus, to screen the LM1212 genes responsible for the regulation of the P₃₅ promoter, the promoter directing the expression of the *cry* gene, the inventors introduced a reporting system into a *Bacillus thuringiensis* strain, as described thereafter.

### 1) Materials and Methods

The Bt strain *kurstaki* HD73 Cry is chosen to done the LM1212 genes responsible for the activation of the P₃₅ promoter and for the negative effect on sporulation.

The thermosensitive plasmid pRN5101-AmyE-HD73::tet is first constructed following the same procedure as described in Verplaetse *et al.,* 2015, except that the antibiotic resistance cassette confers resistance to tetracycline instead of spectinomycin and that the *amyE* flanking regions used for homologous recombination were amplified using Bt HD73 genomic DNA instead of Bt 407. A P_{35'}-*lacZ* transcriptional fusion is then cloned between the *amy* up and *amy* down fragments cloned from the *kurstaki* strain. The P_{35'}*-lacZ* fusion is then introduced at the *amyE* locus of the *kurstaki* HD73 chromosome by homologous recombination (Figure 1).

### 2) Results

The resulting strain is designated HD73 Cry⁻ [amyE::P_{35'}-*lacZ*].

### EXAMPLE 2: CHARACTERIZATION OF THE LM1212 GENES ACTIVATING TRANSCRIPTION FROM THE P₃₅ PROMOTER

### i. Localization of the genes responsible for the expression from the P₃₅ promoter on the DNA of LM1212 strain

### 1) Materials and Methods

The DNA fragment ORF28-ORF32 from plasmid PLM248 of strain LM1212 is cloned into plasmid pHT304 (Arantes, O. *et al.,* 1991) and the resulting plasmid is called pHT-1a. pHT-1a is then introduced into the Bt strain HD73 Cry⁻ [*amyE::*P_{35'}-*lacZ*] and the recombinant clones are isolated on HCT plates (0.7% casein hydrolysate, 0.5% tryptone, 0.68% KH2 PO4, 0.012% MgSO4 _7H2 O, 0.00022% MnSO4_ 4H2 O, 0.0014% ZnSO4_7H2 O, 0.008% ferric ammonium citrate, 0.018% CaCl2_4H2 O, 0.3% glucose, pH 7.2) (Lecadet *et al.,* 1980; Verplaetse *et al.,* 2016) containing yeast extract (0,05%), glucose 0.3% and X-Gal (100 µg / mL).

### 2) Results

The parental strain gives white colonies. In sharp contrast, the strain harboring plasmid pHT-la gives blue colonies (Figure 2). This result indicates that the genes responsible for the expression from the P₃₅ promoter are located on the DNA fragment 1a corresponding to LM1212 ORF28 to 32.

### ii. Identification of the genes involved in the transcriptional activity

### 1) Materials and Methods

The DNA region ORF28 to 32 is sub-cloned into the pHT304 plasmid and the resulting plasmids are transformed into the Bt strain HD73 [*amyE*::P_{35'}*-lacZ*] (figure 2).

### 2) Results

It appears that the ORF28 encoding a transcriptional regulator is sufficient to activate P₃₅-directed transcription. However, this activity is obtained if a short DNA fragment located just upstream of the ORF29 is fused upstream from ORF28 (Fragment 1e, figure 2). ORF28 encodes a transcriptional regulator belonging to the family of response regulators, suggesting that it should act as a two-component system in association with a histidine kinase. Such a kinase may be encoded by ORF32. However, results show that ORF28 alone is able to activate the P₃₅ promoter. *In silico* analysis of these genes (ORF28 and 32) indicates that ORF28 is unique and is not found in the available sequences of the bacteria of the *B. cereus* group (about 100 genomes). In contrast, ORF32 is highly conserved among all the bacteria of the *B. cereus* group, including the LM1212.
Thus, ORF28 may function as a response regulator activated by the histidine kinase naturally present in the *kurstaki* HD73 strain.

### iii. Effect of the plasmid pHT-1c on the sporulation of the bacteria

### 1) Materials and Methods

pHT-lc, which corresponds to the plasmid pHT304 carrying the gene ORF28 and a promoter region, is introduced in Bt strain HD73 [*amyE*::P_{35'}-*lacZ*].

### 2) Results

The introduction of pHT-1c in Bt strain HD73 [*amyE*::P_{35'}*-lacZ*] negatively affects sporulation by a 10-fold factor (Table 1).

**Table 1. Effect of the DNA fragment 1c carrying the cpcR gene on the sporulation of B. thuringiensis stain HD73. The results are the mean of two independent experiments. ^{a}The bacteria were grown in liquid HCT medium (containing Yeast Extract 0.05% and Glucose 0.3%) at 30°C. 48 h after inoculation, aliquots were heated at 80°C for 12 mins. The cells were plated and CFUs corresponding to heat-resistant spores were then counted.**

| **Strains** | **Heat-resistant spores (CFU/ml)^{a}** |
|---|---|
| HD73 *amye*::P*cry*35*-lacZ* (pHT304) | 5,25E+08 (± 5,25E+07) |
| HD73 *amyE*::P*cry35-lacZ* (pHT304-1c) | 4,99E+07 (± 9,42E+06) |

### iv. Conclusion

These data indicate that the gene corresponding to ORF28 encodes the regulator (designated as CpcR) responsible for two functions: i) the activation of *cry* gene expression in the LM1212 strain, and ii) the reduction of the sporulation rate.
The results also suggest that ORF28 functions as a two-component system associated with a kinase existing in all the Bt strains. This gene is responsible for the specific phenotype of the LM1212 strain, which is to differentiate into crystal-producers or spore-formers.

### EXAMPLE 3: USE OF THE CPCR REGULATOR TO PRODUCE CRY1Ab IN NON-SPORULATING BT CELLS

In order to validate the use of CpcR in a Bt strain for reducing its sporulation while maintaining toxin production, the inventors produced recombinant strains of Bt and analyzed the sporulation rate of this recombinant strain and the production of the toxin Cry1Ab.

### 1) Materials and Methods

a) The *cry1Ab* gene, a typical sporulation-dependent *cry* gene is PCR amplified from strain *kurstaki* HD1 (the biopesticide *kurstaki* HD1 Dipel®) and is cloned downstream from the P₃₅ promoter into plasmid pHT16-18 (Lereclus *et al.,* 1992). The resulting plasmid, pHT16-18Ω P35'*-cry1Ab* (*i.e.* the plasmid pHT16-18 carrying the *cry1Ab* gene from the *kurstaki* HD1 Dipel® strain under the control of the P₃₅ promoter) is transformed into Bt strain HD73 Cry⁻. The resulting strain was then transformed with the plasmid pHT-1c carrying the *cpcR* gene. The bacteria were plated on HCT agar plates for 48 h at 30°C and examined in phase-contrast microscopy (Figure 3).
b) The proteins produced by these recombinant strains are analyzed by western blotting with antisera against the Cry1Ab toxin (Figure 4).

### 2) Results

a) The results show the production of typical crystal inclusion in non-sporulating cells of the *kurstaki* strain harboring plasmids pHT16-18.ΩP_{35'}-cry1Ab and pHT-1c. Moreover, the sporulation rate of the recombinant strain carrying pHT16-18ΩP_{35'}-*cry1Ab* and pHT-1c is significantly lower than that of the wild-type strain.
b) The production of Cry1Ab is strongly increased in the *kurstaki* strain harboring the *cpcR* gene and the P₃₅'*-cry1Ab* fusion (Panels A and B, Lanes 3).
   The faint band of Cry1Ab observed in lanes 2 results from a low CpcR-independent expression of *cry1Ab.*

These results show that the invention provides a new range of biopesticides by the way of production of insecticidal Cry toxins in non-sporulating Bt strains.

### EXAMPLE 4: TRANSCRIPTION OF THE P₃₅ PROMOTER IS HIGHER WHEN CLONED ON THE SAME PLASMID AS CPCR

### 1) Materials and Methods

The DNA fragment designated 5a and comprised of ORF28, the intergenic region between ORF28 and ORF29 and the intergenic region between ORF29 and ORF30, was either cloned upstream from a transcriptional fusion between P₃₅ and the reporter gene *lacZ* on vector pHT304.18 (which has the same skeleton as vector pHT304, but the cloning site is in the opposite orientation to that of vector pHT304), giving plasmid pHT-5a-P35'Z, or alone on vector pHT1618, giving plasmid pHT1618-5a. The latter was transformed in Bt strain HD73 Cry⁻ along with pHT- P35'Z, giving strain HD (pHT1618-5a) (pHT-P35'Z). Plasmid pHT-5a-P35'Z was also transformed in Bt strain HD73 Cry⁻, giving strain HD (pHT-5a-P35'Z). The cells were grown in liquid HCT containing 0,05% yeast extract and 0.3% glucose. Samples were harvested and assayed for β-galactosidase activity (Perchat *et al.,* 2011).

### 2) Results

Figure 5 shows that even though P35 is active in both strain HD (pHT1618-5a) (pHT-P35'Z) (light grey squares) and HD (pHT-5a-P35'Z) (dark grey lozenges), its activity is higher in strain HD (pHT-5a-P35'Z), in particular it is 14-fold higher after 4 hours (1200 units versus 17000 units, respectively).

### REFERENCES

Agaisse, H. et al., J. Bacteriol. 1995. 177: 6027-6032.
Arantes, O. et al., Gene 1991. 108: 115-119.
Bone, EJ. et al., FEMS Microbiol Lett. 1989. 49(2-3):171-177.
Bravo A. et al., Insect Biochem Mol Biol. 2011. 41: 423-431.
Crickmore, N. et al., Microbiol. Mol. Biol. Rev. 1998. 62: 807-813.
Deng, C. et al., Toxins 2014. 6: 2194-2209.
Deng C. et al., The ISME Journal. 2015. 9: 286-296.
Fan Q. et al., Acta Microbiol. Sinica. 2017. 57(1):121-130.
Fromm et al., Nature 1990. 319:791-793.
Koehler TM, et al. J Bacteriol. 1994. 176(3):586-595.
Lecadet M et al., Microbiology. 1980. 121: 1: 203-212.
Lereclus, D. et al., FEMS Microbiol. Lett. 1989. 60: 211-217.
Lereclus, D. et al., Nature Biotechnology 1992. 10: 418-421.
Lereclus, D. et al., Mol. Microbiol. 1992. 7: 35-46.
Mahillon, J. et al., Eynard & Teissie (Eds.) Springer Lab. Manual 1999. 242-252.
Peng, D. H., et al. J. Appl. Microbiol. 2009. 106:1849-1858
Perchat S. et al., Molecular microbiology. 2011. 82: 619-633
Sanahuja G. et al., Plant Biotechnol Journal. 2011. 9:283-300.
Schnepf, E. et al., Microbiol. Mol. Biol. Rev. 1998. 62: 775-806.
Song Z.-r. et al., J. Integr. Agricult.. 2017. 16(1):135-143.
Trieu-Cuot, P. et al., FEMS Microbiol Lett 1987. 48: 289-294.
Van Frankenhuizen, K. et al., J. Invertebr. Pathol. 2009. 101 : 1-16.
Verplaetse, E. et al., MBio 2015. 6: e136008-15.
Verplaetse E. et al., Res Microbiol. 2016. doi: 10.1016/j.resmic.2016.03.006.
Wang et al., Acta Microbiol. Sinica. 2010. 50(11):1550-1555.
Wei J-Z. et αl., PNAS 2003. 100(5):2760-2765.

## Claims

1. Use of the *cpcR* regulator gene of the sequence SEQ ID NO: 1 for reducing the sporulation of a recombinant strain of *Bacillus thuringiensis.*

2. The use of the *cpcR* gene as claimed in claim 1, wherein said CpcR regulator directs the expression of promoters of genes encoding Cry and Cyt proteins, said promoters having the sequence SEQ ID NO: 2 as follows:
X₁TGAAX₂AAAAX₃X₄X₅X₆CAX₇X₈AX₉ATTTX₁₀CX₁₁TCX₁₂X₁₃X₁₄X₁₅X₁₆ TX₁₇X₁₈AX₁₉ATGTX₂₀X₂₁TX₂₂GX₂₃TAX₂₄AX₂₅TX₂₆X₂₇X₂₈X₂₉AX₃₀X₃₁TX₃₂ X₃₃, with: X₁=A or G; X₂ =C or T; X₃=A or T; X₄=A, T or G; X₅=A, C or T; X₆=A or G; X₇=C or T; X₈=A or C X₉=A, T or G; X₁₀=A or C; X₁₁= A or C; X₁₂=A, C or T; X₁₃=A, G or T; X₁₄=A or C; X₁₅=A, G or T; X₁₆=A or G; X₁₇=A or T; X₁₈=A, C or T; X₁₉=C or T; X₂₀=A or C; X₂₁=A, C or G; X₂₂=A or T; X₂₃= C or T; X₂₄=G or T; X₂₅=C or T; X₂₆=G or T; X₂₇=A or G; X₂₈=A or T; X₂₉=A, G or T; X₃₀=C, G or T; X₃₁=A or G; X₃₂=A or G; X₃₃=C or T.

3. The use of the *cpcR* gene as claimed in claim 2, **characterized in that** the promoter is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID NO: 6.

4. The use of the *cpcR* gene as claimed in any one of claims 1 to 3, **characterized in that** the genes encoding toxins are *cry* genes or *cyt* genes.

5. The use of the *cpcR* gene as claimed in any one of claims 1 to 4, **characterized in that** the strain of *Bacillus* comprises *cryl, cry2, cry3, cry4, cry5, cry6, cry8, cry9, cry11, cry14, cry21, cyt1* or *cyt2* genes.

6. A recombinant strain of *Bacillus thuringiensis* **characterized in that** it comprises:
a) At least one gene encoding Cry and/or Cyt toxins,
b) At least one promoter having the sequence SEQ ID NO: 2, allowing the expression of said at least one gene encoding Cry and/or Cyt toxins, and,
c) A *cpcR* regulator gene of sequence SEQ ID NO: 1, which directs the expression of said at least one promoter.

7. The recombinant strain of *Bacillus thuringiensis* as claimed in claim 6, **characterized in that** the promoter is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID NO: 6.

8. The recombinant strain of *Bacillus thuringiensis* as claimed in claim 6 or in claim 7, **characterized in that** the promoter is cloned on the same plasmid as the *cpcR* regulator gene.

9. The recombinant strain of *Bacillus thuringiensis* as claimed in any one of claims 6 to 8, **characterized in that** the genes encoding toxins are *cry* genes or *cyt* genes.

10. The recombinant strain of *Bacillus thuringiensis* as claimed in any one of claims 6 to 9, **characterized in that** the strain of *Bacillus* comprises *cry1, cry2, cry3, cry4, cry5, cry6, cry8, cry9, cry11, cry14, cry21, cyt1* or *cyt2* genes.

11. Use of a recombinant strain of *Bacillus thuringiensis* as claimed in anyone of claims 6-10 as biopesticide, wherein said use is not a method for treatment of the human or animal body by therapy.

12. The use of the recombinant strain of *Bacillus thuringiensis* as claimed in claim 11 for protecting cultures, wherein said use is not a method for treatment of the human or animal body by therapy.

13. The use of the recombinant strain of *Bacillus thuringiensis* as claimed in claim 11 to control vectors, especially vectors that transmit pathogens responsible for mammalian diseases, such as insect vectors, in particular mosquitoes or simuliid , wherein said use is not a method for treatment of the human or animal body by therapy.

14. The use of the recombinant strain of *Bacillus thuringiensis* as claimed in claim 11 to control nematodes, especially those responsible for mammalian diseases, wherein said use is not a method for treatment of the human or animal body by therapy.

15. A method for obtaining a recombinant strain of *Bacillus thuringiensis,* comprising the steps of introducing in said strain both:
1- a genetic construction comprising at least one gene encoding a toxin under the control of a promoter having the sequence SEQ ID NO: 2, and
2- an expression system comprising the *cpcR* regulator gene of the sequence SEQ ID NO: 1.

16. The method for obtaining a recombinant strain of *Bacillus thuringiensis,* as claimed in claim 15, wherein the genetic construction as defined in step 1 of claim 15 and the expression system as defined in step 2 of claim 15 are on the same plasmid.

## Patentansprüche

1. Verwendung des *cpcR*-Regulatorgens der Sequenz SEQ ID NO: 1 zum Verringern der Sporenbildung eines rekombinanten Stamms von *Bacillus thuringiensis.*

2. Verwendung des *cpcR-Gens* nach Anspruch 1, wobei der CpcR-Regulator die Exprimierung von Promotoren von Genen steuert, die für Cry- und Cyt-Proteine codieren, wobei die Promotoren die Sequenz SEQ ID NO: 2 aufweisen wie folgt:
X₁TGAAX₂AAAAX₃X₄X₅X₆CAX₇X₈AX₉ATTTX₁₀CX₁₁TCX₁₂X₁₃X₁₄X_{1 5}X₁₆TX₁₇X₁₈AX₁₉ATGTX₂₀X₂₁TX₂₂GX₂₃TAX₂₄AX₂₅TX₂₆X₂₇X₂₈X₂₉AX₃₀X₃₁TX₃₂X₃₃, mit: X₁ = A oder G; X₂ = C oder T; X₃ = A oder T; X₄ = A, T oder G; X₅ = A, C oder T; X₆ = A oder G; X₇ = C oder T; X₈ = A oder C; X₉ = A, T oder G; X₁₀ = A oder C; X₁₁ = A oder C; X₁₂ = A, C oder T; X₁₃ = A, G oder T; X₁₄ = A oder C; X₁₅ = A, G oder T; X₁₆ = A oder G; X₁₇ = A oder T; X₁₈ = A, C oder T; X₁₉ = C oder T; X₂₀ = A oder C; X₂₁ = A, C oder G; X₂₂ = A oder T; X₂₃ = C oder T; X₂₄ = G oder T; X₂₅ = C oder T; X₂₆ = G oder T; X₂₇ = A oder G; X₂₈ = A oder T; X₂₉ = A, G oder T; X₃₀ = C, G oder T; X₃₁ = A oder G; X₃₂ = A oder G; X₃₃ = C oder T.

3. Verwendung des *cpcR-Gens* nach Anspruch 2, **dadurch gekennzeichnet, dass** der Promotor ausgewählt ist aus der Gruppe, bestehend aus: P₃₂ der Sequenz SEQ ID NO: 3, P₄₁ der Sequenz SEQ ID NO: 4, P₃₅ der Sequenz SEQ ID NO: 5, P₄₅ der Sequenz SEQ ID NO: 6.

4. Verwendung des *cpcR-Gens* nach einem der Ansprüche 1 bis 3, **dadurch gekennzeichnet, dass** die Gene, die für Toxine codieren, *cry*-Gene oder *cyt-*Gene sind.

5. Verwendung des *cpcR-Gens* nach einem der Ansprüche 1 bis 4, **dadurch gekennzeichnet, dass** der Stamm von *Bacillus cry1-, cry2-, cry3-, cry4-, cry5-, cry6-, cry8-, cry9-, cry11-, cry14-, cry21-, cyt1-* oder *cyt2-Gene* umfasst.

6. Rekombinanter Stamm von *Bacillus thuringiensis,* **dadurch gekennzeichnet, dass** er umfasst:
a) mindestens ein Gen, das für Cry- und/oder Cyt-Toxine codiert,
b) mindestens einen Promotor mit der Sequenz SEQ ID NO: 2, der die Exprimierung des mindestens einen Gens, das für Cry- und/oder Cyt-Toxine codiert, erlaubt, und
c) ein *cpcR*-Regulatorgen der Sequenz SEQ ID NO: 1, das die Exprimierung des mindestens einen Promotors steuert.

7. Rekombinanter Stamm von *Bacillus thuringiensis* nach Anspruch 6, **dadurch gekennzeichnet, dass** der Promotor ausgewählt ist aus der Gruppe, bestehend aus: P₃₂ der Sequenz SEQ ID NO: 3, P₄₁ der Sequenz SEQ ID NO: 4, P₃₅ der Sequenz SEQ ID NO: 5, P₄₅ der Sequenz SEQ ID NO: 6.

8. Rekombinanter Stamm von *Bacillus thuringiensis* nach Anspruch 6 oder Anspruch 7, **dadurch gekennzeichnet, dass** der Promotor auf dem gleichen Plasmid geklont ist wie das *cpcR*-Regulatorgen.

9. Rekombinanter Stamm von *Bacillus thuringiensis* nach einem der Ansprüche 6 bis 8, **dadurch gekennzeichnet, dass** die Gene, die für Toxine codieren, *cry*-Gene oder *cyt-*Gene sind.

10. Rekombinanter Stamm von *Bacillus thuringiensis* nach einem der Ansprüche 6 bis 9, **dadurch gekennzeichnet, dass** der Stamm von *Bacillus cry1-, cry2-, cry3-, cry4-, cry5-, cry6-, cry8-, cry9-, cry11-, cry14-, cry21-, cyt1-* oder *cyt2-*Gene umfasst.

11. Verwendung eines rekombinanten Stamms von *Bacillus thuringiensis* nach einem der Ansprüche 6-10 als Biopestizid, wobei die Verwendung kein Verfahren zur Behandlung des menschlichen oder tierischen Körpers durch Therapie ist.

12. Verwendung des rekombinanten Stamms von *Bacillus thuringiensis* nach Anspruch 11 zum Schützen von Kulturen, wobei die Verwendung kein Verfahren zur Behandlung des menschlichen oder tierischen Körpers durch Therapie ist.

13. Verwendung des rekombinanten Stamms von *Bacillus thuringiensis* nach Anspruch 11, um Vektoren zu kontrollieren, insbesondere Vektoren, die Pathogene übertragen, die verantwortlich sind für Krankheiten bei Säugetieren, wie Insektenvektoren, besonders Moskitos oder schwarze Fliege, wobei die Verwendung kein Verfahren zur Behandlung des menschlichen oder tierischen Körpers durch Therapie ist.

14. Verwendung des rekombinanten Stamms von *Bacillus thuringiensis* nach Anspruch 11, um Nematoden zu kontrollieren, insbesondere jene, die für Krankheiten bei Säugetieren verantwortlich sind, wobei die Verwendung kein Verfahren zur Behandlung des menschlichen oder tierischen Körpers durch Therapie ist.

15. Verfahren zum Erhalten eines rekombinanten Stamms von *Bacillus thuringiensis,* umfassend die Schritte des Einführens in den Stamm von sowohl:
1- einem genetischen Konstrukt, umfassend mindestens ein Gen, das ein Toxin unter der Kontrolle eines Promotors mit der SEQ ID NO: 2 codiert, als auch
2- eines Exprimierungssystems, umfassend das *cpcR-*Regulatorgen der Sequenz SEQ ID NO: 1.

16. Verfahren zum Erhalten eines rekombinanten Stamms von *Bacillus thuringiensis* nach Anspruch 15, wobei das genetische Konstrukt wie in Schritt 1 von Anspruch 15 definiert und das Expressionssystem wie in Schritt 2 von Anspruch 15 definiert auf dem gleichen Plasmid vorliegen.

## Revendications

1. Utilisation du gène régulateur *cpcR* de la séquence SEQ ID NO : 1 pour réduire la sporulation d'une souche recombinante de *Bacillus thuringiensis.*

2. Utilisation du gène *cpcR* selon la revendication 1, dans laquelle ledit régulateur CpcR dirige l'expression de promoteurs de gènes codant des protéines Cry et Cyt, lesdits promoteurs ayant la séquence SEQ ID NO : 2 comme suit :
X₁TGAAX₂AAAAX₃X₄X₅X₆CAX₇X₈AX₉ATTTX₁₀CX₁₁TCX₁₂X₁₃X₁₄X₁₅ X₁₆TX₁₇X₁₈AX₁₉ATGTX₂₀X₂₁TX₂₂GX₂₃TAX₂₄AX₂₅TX₂₆X₂₇X₂₈X₂₉AX₃₀X₃₁TX₃₂X₃₃, avec : X1=A ou G ; X₂=C ou T ; X₃=A ou T ; X₄=A, T ou G ; X₅=A, C ou T ; X₆=A ou G ; X₇=C ou T ; X₈=A ou C ; X₉=A, T ou G ; X₁₀=A ou C ; X₁₁= A ou C ; X₁₂=A, C ou T ; X₁₃=A, G ou T ; X₁₄=A ou C ; X₁₅=A, G ou T ; X₁₆=A ou G ; X₁₇=A ou T ; X₁₈=A, C ou T ; X₁₉=C ou T ; X₂₀=A ou C ; X₂₁=A, C ou G ; X₂₂=A ou T ; X₂₃= C ou T ; X₂₄=G ou T ; X₂₅=C ou T ; X₂₆=G ou T ; X₂₇=A ou G ; X₂₈=A ou T ; X₂₉=A, G ou T ; X₃₀=C, G ou T ; X₃₁=A ou G ; X₃₂=A ou G ; X₃₃=C ou T.

3. Utilisation du gène *cpcR* selon la revendication 2, **caractérisée en ce que** le promoteur est sélectionné à partir du groupe consistant en : P₃₂ de la séquence SEQ ID NO : 3, P₄₁ de la séquence SEQ ID NO : 4, P₃₅ de la séquence SEQ ID NO : 5, P₄₅ de la séquence SEQ ID NO : 6.

4. Utilisation du gène *cpcR* selon l'une quelconque des revendications 1 à 3, **caractérisée en ce que** les gènes codant des toxines sont des gènes *cry* ou des gènes *cyt.*

5. Utilisation du gène *cpcR* selon l'une quelconque des revendications 1 à 4, **caractérisée en ce que** la souche de *Bacillus* comprend des gènes *cry1, cry2, cry3, cry4, cry5, cry6, cry8, cry9, cry11, ery14, cry21, cyt1* ou *cyt2.*

6. Souche recombinante de *Bacillus thuringiensis* **caractérisée en ce qu'**elle comprend :
a) Au moins un gène codant des toxines Cry et/ou Cyt,
b) Au moins un promoteur ayant la séquence SEQ ID NO : 2, permettant l'expression dudit au moins un gène codant des toxines Cry et/ou Cyt, et,
c) Un gène régulateur *cpcR* de séquence SEQ ID NO : 1, qui dirige l'expression dudit au moins un promoteur.

7. Souche recombinante de *Bacillus thuringiensis* selon la revendication 6, **caractérisée en ce que** le promoteur est sélectionné à partir du groupe consistant en : P₃₂ de la séquence SEQ ID NO :3, P₄₁ de la séquence SEQ ID NO : 4, P₃₅ de la séquence SEQ ID NO : 5, P₄₅ de la séquence SEQ ID NO : 6.

8. Souche recombinante de *Bacillus thuringiensis* selon la revendication 6 ou la revendication 7, **caractérisée en ce que** le promoteur est cloné sur le même plasmide que le gène régulateur *cpcR.*

9. Souche recombinante de *Bacillus thuringiensis* selon l'une quelconque des revendications 6 à 8, **caractérisée en ce que** les gènes codant des toxines sont des gènes *cry* ou des gènes *cyt.*

10. Souche recombinante de *Bacillus thuringiensis* selon l'une quelconque des revendications 6 à 9, **caractérisée en ce que** la souche de *Bacillus* comprend des gènes *cry1, cry2, cry3, cry4, cry5, cry6, cry8, cry9, cry11, cry14, cry21, cyt1* ou *cyt2.*

11. Utilisation d'une souche recombinante de *Bacillus thuringiensis* selon l'une quelconque des revendications 6-10 comme biopesticide, dans laquelle ladite utilisation n'est pas une méthode de traitement du corps humain ou animal par thérapie.

12. Utilisation de la souche recombinante de *Bacillus thuringiensis* selon la revendication 11 pour la protection de cultures, dans laquelle ladite utilisation n'est pas une méthode de traitement du corps humain ou animal par thérapie.

13. Utilisation de la souche recombinante de *Bacillus thuringiensis* selon la revendication 11 pour contrôler des vecteurs, en particulier des vecteurs transmettant des agents pathogènes responsables de maladies de mammifères, tels que des vecteurs insectes, en particulier des moustiques ou simulie, dans laquelle ladite utilisation n'est pas une méthode de traitement du corps humain ou animal par thérapie.

14. Utilisation de la souche recombinante de *Bacillus thuringiensis* selon la revendication 11 pour contrôler des nématodes, en particulier ceux responsables de maladies de mammifères, dans laquelle ladite utilisation n'est pas une méthode de traitement du corps humain ou animal par thérapie.

15. Procédé d'obtention d'une souche recombinante de *Bacillus thuringiensis,* comprenant les étapes d'introduction dans ladite souche à la fois de :
1- une construction génétique comprenant au moins un gène codant une toxine sous le contrôle d'un promoteur ayant la séquence SEQ ID NO : 2, et
2- un système d'expression comprenant le gène régulateur *cpcR* de la séquence SEQ ID NO : 1.

16. Procédé d'obtention d'une souche recombinante de *Bacillus thuringiensis* selon la revendication 15, dans lequel la construction génétique telle que définie à l'étape 1 de la revendication 15 et le système d'expression tel que défini à l'étape 2 de la revendication 15 sont sur le même plasmide.


### SEQUENCE LISTING

<110> INSTITUT NATIONAL DE LA RECHERCHE AGRONOMIQUE
   INSTITUTE OF PLANT PROTECTION, CHINESE ACADEMY OF
   AGRICULTURAL SCIENCES
   LERECLUS, Didier
   SLAMTI, Leyla
   SONG, Fu Ping
   DENG, Chao
   ZHANG, Jie
<120> Use of the CpcR regulator gene for obtaining new recombinant strains of Bacillus thuringiensis with reduced sporulation capacity
<130> F539/186
<150> EP17305011.3
   <151> 2017-01-05
<160> 6
<170> PatentIn version 3.5
<210> 1
   <211> 663
   <212> DNA
   <213> Bacillus thuringiensis
<400> 1
   atgaatccta atatacttgt gataaatggt aatacgaaga atcaagaatt tattcaaaat    60
   ctcctgatta taaagaaatt catgattgat acggtaaatg atggtataga aggaattatt   120
   cgctttcaaa agaaagtgta cgatctagtt atactagatg ttatgtcacc taatttagat   180
   ggatttagta tatgtaaaat tataagatca caatctaaag tgccaattat catgttatct   240
   acaataaagg acgaaagtat tgagattaaa ggatttcaat ttggtattga tgactttatt   300
   actctaccat gttcagttga gctattttat tataggatag aagccattct acgaggacga   360
   aattcaactg attcattgtc attaatacag tttcaagaaa tatcactaaa tcctgattcg   420
   tatatagtgt atcttaatgg gcagaagaaa aagctaacga caaaagaatt tgatatgcta   480
   catatatttt taagaaatcc agggaaagta ttatctagag aatttttgct gaatcaagtg   540
   tggggatatg attattatgg agatccacga gtcatagatg cgtatataaa aaaactacgc   600
   aaaaagttaa gtattccgta tataaaaaca ataacaggcg ttggttataa actagacaca   660
   taa                                                                 663
<210> 2
   <211> 65
   <212> DNA
   <213> Artificial Sequence
<220>
   <223> Promoter activated by CpcR consensus sequence
<220>
   <221> Variant
   <222> (1)..(1)
   <223> Replace = a
<220>
   <221> Variant
   <222> (6).. (6)
   <223> Replace = t
<220>
   <221> Variant
   <222> (11)..(11)
   <223> Replace = t
<220>
   <221> Variant
   <222> (12)..(12)
   <223> Replace = t or g
<220>
   <221> Variant
   <222> (13)..(13)
   <223> Replace = c or t
<220>
   <221> Variant
   <222> (14)..(14)
   <223> Replace = a
<220>
   <221> Variant
   <222> (17)..(17)
   <223> Replace = t
<220>
   <221> Variant
   <222> (18)..(18)
   <223> Replace = c
<220>
   <221> Variant
   <222> (20)..(20)
   <223> Replace = t or g
<220>
   <221> Variant
   <222> (25)..(25)
   <223> Replace = a
<220>
   <221> Variant
   <222> (27)..(27)
   <223> Replace = c
<220>
   <221> Variant
   <222> (30)..(30)
   <223> Replace = c or t
<220>
   <221> Variant
   <222> (31)..(31)
   <223> Replace = a or g
<220>
   <221> Variant
   <222> (32)..(32)
   <223> Replace = c
<220>
   <221> Variant
   <222> (33)..(33)
   <223> Replace = g or t
<220>
   <221> Variant
   <222> (34)..(34)
   <223> Replace = g
<220>
   <221> Variant
   <222> (36)..(36)
   <223> Replace = t
<220>
   <221> Variant
   <222> (37)..(37)
   <223> Replace = c or t
<220>
   <221> Variant
   <222> (39)..(39)
   <223> Replace = t
<220>
   <221> Variant
   <222> (44)..(44)
   <223> Replace = c
<220>
   <221> Variant
   <222> (45)..(45)
   <223> Replace = c or g
<220>
   <221> Variant
   <222> (47)..(47)
   <223> Replace = a
<220>
   <221> Variant
   <222> (49)..(49)
   <223> Replace = t
<220>
   <221> Variant
   <222> (52)..(52)
   <223> Replace = t
<220>
   <221> Variant
   <222> (54)..(54)
   <223> Replace = c
<220>
   <221> Variant
   <222> (56)..(56)
   <223> Replace = t
<220>
   <221> Variant
   <222> (57)..(57)
   <223> Replace = g
<220>
   <221> Variant
   <222> (58)..(58)
   <223> Replace = t
<220>
   <221> Variant
   <222> (59)..(59)
   <223> Replace = g or t
<220>
   <221> Variant
   <222> (61)..(61)
   <223> Replace = g or t
<220>
   <221> Variant
   <222> (62)..(62)
   <223> Replace = a
<220>
   <221> Variant
   <222> (64)..(64)
   <223> Replace = g
<220>
   <221> Variant
   <222> (65)..(65)
   <223> Replace = t
<400> 2
   gtgaataaaa aaagcacaaa atttccatca tcagttaaca tgtaattgct agattgaaaa 60
   cgtac 65
<210> 3
   <211> 65
   <212> DNA
   <213> Bacillus thuringiensis
<400> 3
   gtgaacaaaa agtacatcaa atttccatcc tcggtttaca tgtacttgct atattggaga 60
   ggtac 65
<210> 4
   <211> 65
   <212> DNA
   <213> Bacillus thuringiensis
<400> 4
   gtgaacaaaa aacacatcag atttccatct gagatttaca tgtacttgct atattggtaa 60
   ggtac 65
<210> 5
   <211> 65
   <212> DNA
   <213> Bacillus thuringiensis
<400> 5
   gtgaataaaa taagcacaat atttccctca tcagttcata tgtcattgct agactgaaga        60
   tgtat                                                                    65
<210> 6
   <211> 65
   <212> DNA
   <213> Bacillus thuringiensis
<400> 6
   atgaataaaa tttgcacaag atttacatca actataaaca tgtagtagtt atatttaata        60
   catgt                                                                    65